# EUROPEAN PATENT SPECIFICATION

(11) **EP 3 411 366 B1**
(45) Date of publication and mention of the grant of the patent: **23.11.2022**
(21) Application number: 17747059.8
(22) Date of filing: 19.01.2017
(51) Int. Cl.: C07D 401/04, C07D 209/48

(54) **METHOD FOR THE PRODUCTION OF POMALIDOMIDE**
VERFAHREN ZUR HERSTELLUNG VON POMALIDOMID
PROCÉDÉ DE PRODUCTION DE POMALIDOMIDE

(30) Priority: 04.02.2016 HU 1600058
(43) Date of publication of application: 12.12.2018
(73) Proprietor: Egis Gyógyszergyár Zrt., 1106 Budapest (HU)
(72) Inventor: VOLK, Balázs, 1106 Budapest (HU); KÁTAINÉ FADGYAS, Katalin, 2040 Budaörs (HU); LUKÁCS, Gyula, 1163 Budapest (HU); TÓTHNÉ LAURITZ, Mária, 1042 Budapest (HU); DANCSÓ, András, 1183 Budapest (HU); KIRÁLY, Imre, 1076 Budapest (HU); PALOTAI, László, 1239 Budapest (HU); KORMÁNY, Róbert, 4116 Berekböszörmény (HU)
(86) International application number: PCT/HU2017/050002
(87) International publication number: WO 2017/134476

(56) References cited:
- WO-A1-01/34606
- WO-A1-2007/005972
- WO-A1-2015/075694
- US-A1- 2006 160 854
- MULLER G W ET AL: "Amino-substituted thalidomide analogs: potent inhibitors of TNF-@a production", BIOORGANIC & MEDICINAL CHEMISTRY LETTERS, ELSEVIER, AMSTERDAM, NL, vol. 9, no. 11, 7 June 1999 (1999-06-07), pages 1625-1630, XP004169632, ISSN: 0960-894X, DOI: 10.1016/S0960-894X(99)00250-4

## Description

The object of the invention relates to a new, cost-effective, productive method for the production of the pharmaceutical active substance pomalidomide that can be also implemented at industrial scales.

### THE STATE OF THAT ART

With respect to the present invention the state of the art is presented in relation to the following structure 1 *(RS)*-4-amino-2-(2,6-dioxopiperidin-3-yl)isoindole-1,3-dione, with international non-proprietary name pomalidomide.

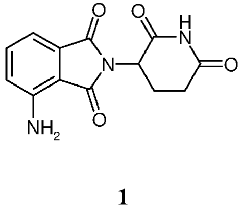

The pomalidomide free base is marketed as the "Form A" disclosed in international patent publication document number WO 2013126326.

It is known that due to it inhibiting angiogenesis and cell growth pomalidomide may be used to good effect in multiple myeloma (plasma cell myeloma) therapy, but human studies are also underway in connection with the treatment of other cancer diseases (myelofibrosis).

Pomalidomide was first described in United States of America patent with disclosure number US 5635517. On the basis of the disclosed examples the steps of the method used are summarised in the following reaction scheme 1.

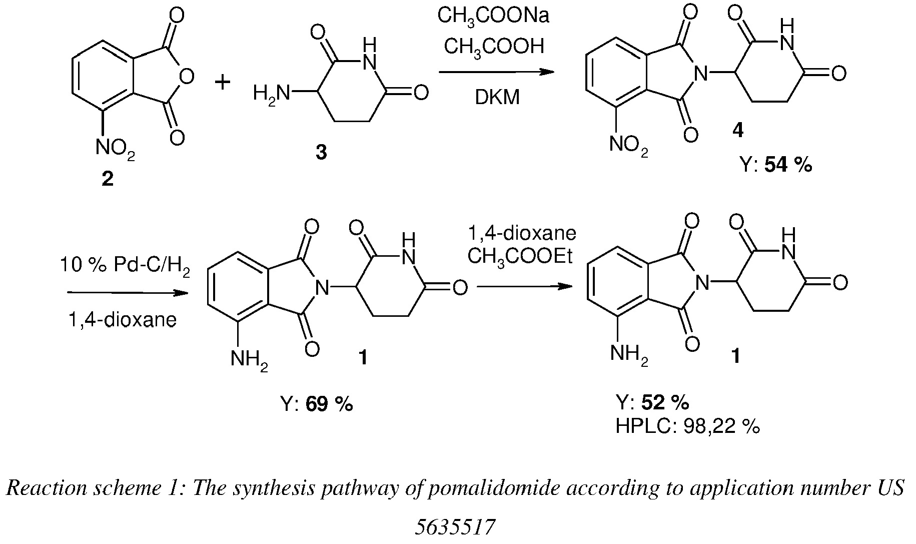

International patent publication document number WO 2002064083 deals with the production of the optically active (R and S) forms of the pomalidomide base. The S-isomer is obtained with the method detailed in reaction scheme 2.

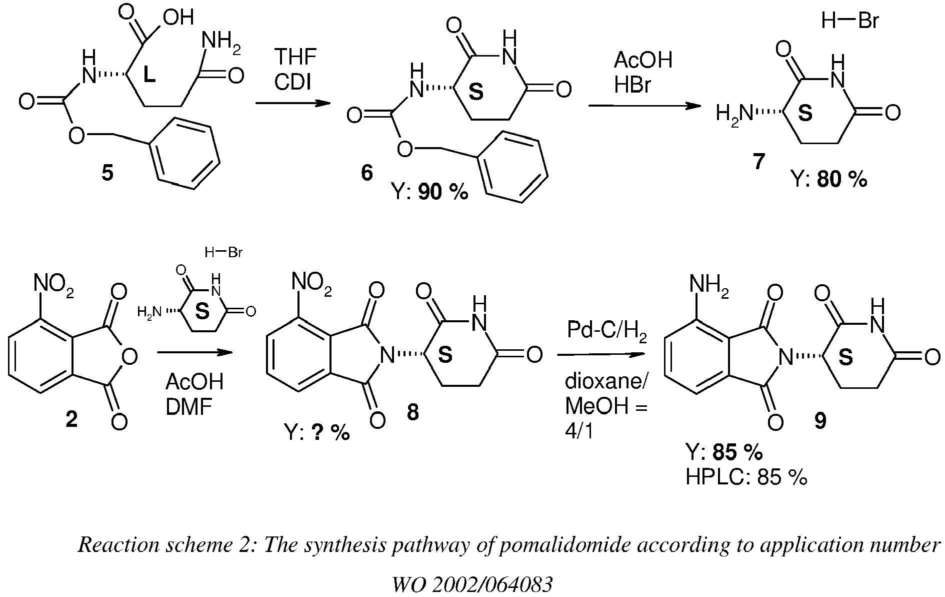

Disregarding the production of the cyclic glutarimide derivative, the method reaches the optically active derivatives in two steps, using the same pathway as that described in the basic patent. The product, assuming 100% quantitative yield of the formula 8 intermediate (3-nitro-thalidomide), is produced at 85% yield and 85% HPLC purity and is characterised by melting point and ¹H-NMR data.

The R-isomer is obtained using a similar method, and using the appropriate D-glutamine, however the total yield and HPLC purity of the latter product are not stated in the application.

US patent application number US 2006160854 also deals with the production of the optically active (R and S) forms of the pomalidomide base. The S-isomer is obtained with the method detailed in reaction scheme 3.

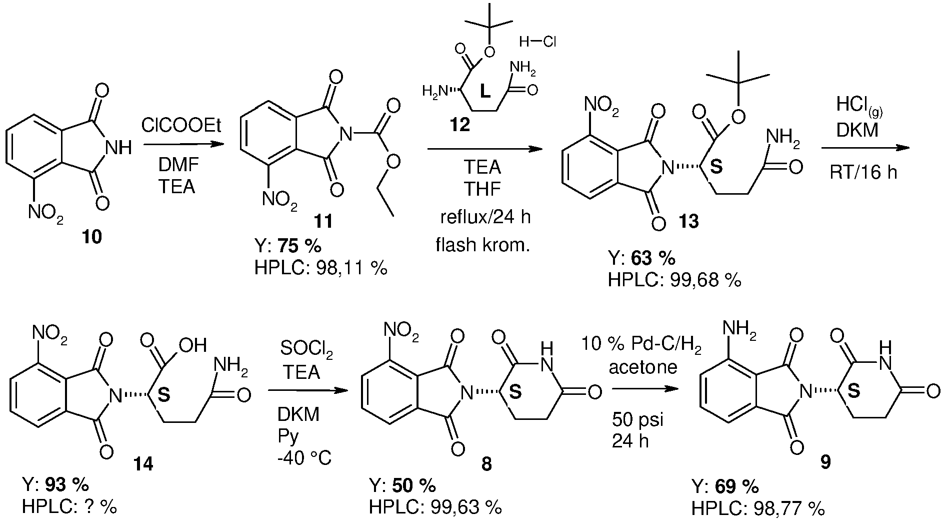

### Reaction scheme 3: The synthesis pathway of pomalidomide according to application number US 2006160854

The method contains numerous steps that are not preferable from the point of view of pharmaceutical industry upscaling (chromatography, long reaction time, low temperature). Using the five steps of the reaction series the active S-isomer is produced with 15% total yield and with 98.77% HPLC purity. In addition to this the product is characterised by melting point, chiral HPLC, ¹H-NMR, ¹³C-NMR and element analysis data.

When producing the R-isomer using a similar method, and the appropriate D-glutamine, the final product is obtained with a total yield of 22% at an HPLC purity level of 99.68%.

US application number US 20070004920 discusses numerous further pomalidomide production methods. Reaction scheme 4 provides an overview of these methods, the reaction arrows also show the example serial number according to the patent application.

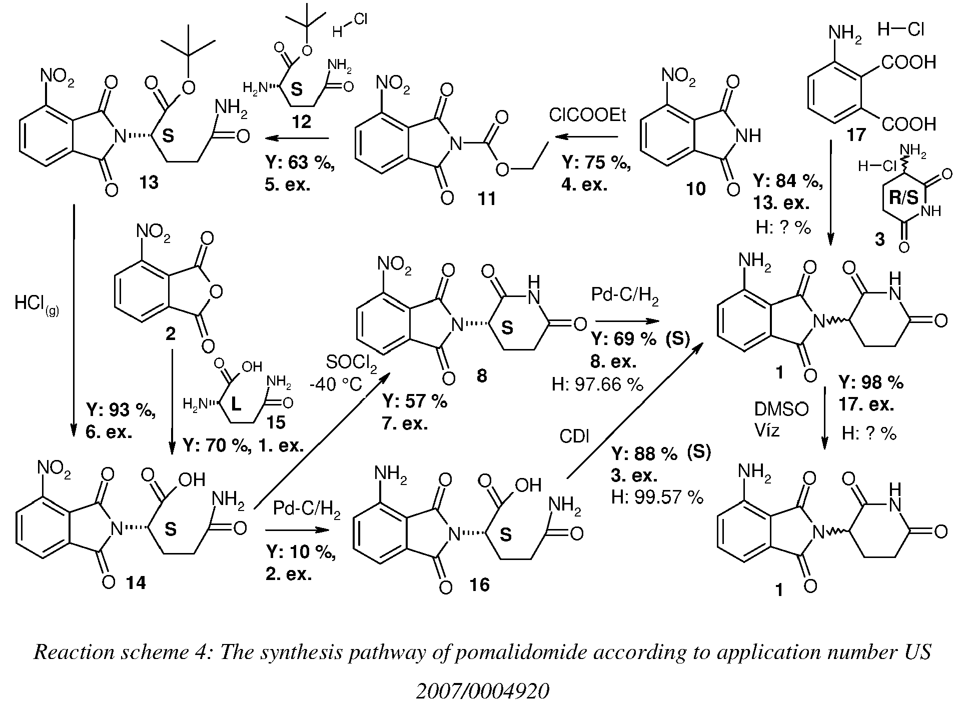

A total of four pomalidomide synthesis pathways can be derived from the described examples, of which two lead to the racemic product, and two others each lead to the optically active S and R isomers. The authors also disclose a recrystallization step for the purification of the raw product obtained in the above methods.

The first reaction pathway implemented at the kilogram-scale (example series 1, 2, 3, reaction series **2** → **14** → **16** → **1,** without recrystallization) provides the racemic product in three steps, with low, 6% yield and 99.57% purity. In addition the product was characterised by melting point, ¹H-NMR, ¹³C-NMR and element analysis.

The synthesis series used for the production of the optically active S-isomer (example series 4, 5, 6, 7, 8, reaction series **10 → 11 → 13 → 14 → 8 → 1)** provides the product in five steps with a total yield of 17% and 98.77% HPLC purity. In addition the product was characterised by melting point, chiral HPLC, ¹H-NMR, ¹³C-NMR and element analysis data.

Only the synthesis of the S-isomer can be seen in the illustrative reaction scheme, the production of its enantiomer takes place analogously using D-glutamine (example series 4, 9, 10, 11, 12 in the application), which results in the R-isomer in five steps, with 22% yield and 97.48% HPLC purity. In addition the product was characterised by melting point, chiral HPLC, ¹H-NMR, ¹³C-NMR and element analysis data.

The last reaction pathway resulting in the racemic product reaches the final product in one step. One of the initial substances of the method, 3-aminophthalic acid hydrochloride, (**17,** reaction scheme 4) has only limited commercial availability, and as compared to the components forming the subject of the present application its price is nearly an order of magnitude higher. The authors mention several versions for the production of the product from this latter component (examples 13, 14, 15, 16), among which one (example 13, reaction **17 → 1)** involves the processing of the reaction mixture and the isolation of the product. The yield relating to the isolated, raw product is 84%, concluding from the examples (example 17) this is recrystallized at a yield level of 98% and is characterised by melting point. Neither the raw product nor the recrystallized product are analytically classified, therefore it does not turn out why the recrystallization was necessary nor whether the product obtained after this complies with the requirements set down in the pharmacopoeia.

International patent application number WO 01034606 in example 30 and example 35 disclose compound 20 of the present invention, ie. 3-amino-N-ethoxycarbonylphtalimide as a starting material for the preparation of different isoindoline derivatives.

International patent application number WO 2007005972 relates to the process of the preparation of pomalidomide, wherein 3-aminophthalic acid hydrochloride is used as intermediate (see example 13-16).

International patent application number WO 2015075694 also relates to the process for the preparation of pomalidomide.

### THE OBJECTIVE OF THE INVENTION

Methods used in the pharmaceuticals industry must meet numerous requirements. The active substances obtained via the method must have the purity specified in the ICH guidelines, i.e. the amount of the contaminant components of unknown structure may not exceed 0.10%, the amount of non-toxic contaminants, and contaminants of known structure may not exceed 0.15%, and the amounts of inorganic substances, heavy metals, solvent residues may not exceed the prescribed limit values either. In addition to this every single step of the technology used must be simple to implement, reproducible from the point of view of product/intermediate quality/quantity, upscalable and, last but not least, cost effective. Accordingly, effective methods typically do not include complete solvent removal, lyophilisation, chromatography or other low-yield and costly operations.

The objective of the elaboration of the invention is the production of the commercially available form of pomalidomide (Form A) using a novel method that provides the isolated product at a high level of purity, at the same yield level as that stated in the literature, or higher and that is reproducible and may be used at the industrial scale in a cost-effective way. The prior art does not include such a solution.

### THE BRIEF DESCRIPTION OF THE INVENTION

As a result of our efforts according to the objective of the invention 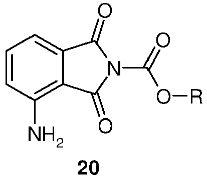 the production method of the compound of general formula **20.**

The object of the invention also relates to a method for the production of formula **1** pomalidomide or its pharmaceutically acceptable salts, hydrates and cocrystals using the compound of general formula **20.**

Accordingly the object of the invention relates to a method for the production of the pomalidomide pharmaceutical active substance in a novel, productive way that may also be implemented at industrial scales.

The object of the invention relates to a method for the production of compound **20,** wherein the formula R means
- a 1-6 carbon atom straight or branched chain, or 3-6 carbon atom cyclic saturated or partially or completely unsaturated hydrocarbon group, or
- a 1-6 carbon atom straight or branched chain, or 3-6 carbon atom cyclic saturated or partially or completely unsaturated, partially or completely halogenated hydrocarbon group, or
- an aryl group, which may be substituted with a 1-6 carbon atom straight or branched chain, saturated or partially or completely unsaturated hydrocarbon group, which, optionally, is partially or completely halogenated, or with a halogen atom, or
- a benzyl group that may contain substituents in the aromatic ring.
   where compound **18** 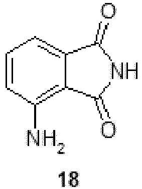 or its acid addition salt is reacted with compound **19**

   ClCOOR **19**

   in the presence of a base; or a metal salt of compound **18** is reacted with compound **19** optionally in the presence of a base, optionally in a further solvent at a temperature between (- 20) - 50 °C, preferably between 0-25 °C, especially preferably between 0-5 °C, where in compound **19** R means
- a 1-6 carbon atom straight or branched chain saturated or partially or completely unsaturated hydrocarbon group,
- a 1-6 carbon atom straight or branched chain saturated or partially or completely unsaturated, partially or completely halogenated hydrocarbon group, or
- an aryl group, which may be substituted with a 1-6 carbon atom straight or branched chain, saturated or partially or completely unsaturated hydrocarbon group, which, optionally, is partially or completely halogenated, or with a halogen atom,
- a benzyl group that may contain substituents in the aromatic ring.

The object of the invention also relates to a method for the production of compound **20,** where in formula **19** R preferably means a 1-6 carbon atom straight or branched chain saturated or partially or completely unsaturated hydrocarbon group, or benzyl group, preferably a methyl, ethyl or benzyl group, especially preferably an ethyl group.

The object of the invention also relates to a method for the production of compound **20,** where the base used is pyridine or a derivative of it, or an aliphatic amine, within this a straight or branched chain, ring tertiary or secondary amine, or salts of alkali metals formed with organic acids, preferably pyridine, diisopropylethylamine, triethylamine, morpholine, Na-acetate, diazabicyclooctane, especially preferably triethylamine or diisopropylethylamine.

The object of the invention also relates to a method for the production of formula **1** pomalidomide or its pharmaceutically acceptable salts, hydrates and cocrystals, where the general formula compound **20** is reacted with glutamine, its open chain or cyclic derivative or the metal salt of any of these, preferably with compound **3** or its metal salt, optionally in the presence of an acid binder; or glutamine, with its acid addition salt of an open chain or cyclic derivative, preferably with the acid addition salt of compound **3** 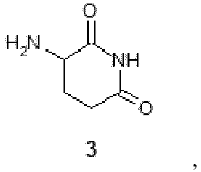 in the presence of an acid binder, optionally in further solvent and then recrystallized as necessary.

The object of the invention also relates to a method for the production of pomalidomide, where the compound of general formula **20** is reacted with the hydrochloride salt of compound **3.**

The object of the invention also relates to a method for the production of pomalidomide, where the acid binder used is pyridine or its derivative, or an aliphatic amine, within this a straight or branched chain, cyclic tertiary or secondary amine, or a salt of alkali metals formed with organic acids, preferably pyridine, diisopropylethylamine, triethylamine, morpholine, Na-acetate, especially preferably triethylamine or Na-acetate.

### A DETAILED DESCRIPTION OF THE INVENTION

### DEFINITIONS

In the context of the present invention the following expressions have been used with the following meanings.

Aryl group: a functional group derivable from a simple aromatic ring.

Salts of compound **18:** the metal salt of compound **18** formed with potassium.

Benzyl group substituents: the substituents are not especially limited, however 4-nitro and 2-chlorobenzyl compounds are highlighted.

Glutamine derivatives: the derivatives of glutamine are not especially limited, suitable derivatives may be, for example, isoglutamine, t-butyl glutamate or benzyl glutamate.

The object of our invention relates to the production method of the compound group of general formula **20,** where in the formula R means
- a 1-6 carbon atom straight or branched chain, or 3-6 carbon atom cyclic, saturated or partially or completely unsaturated hydrocarbon group, or
- a 1-6 carbon atom straight or branched chain, or 3-6 carbon atom cyclic, saturated or partially or completely unsaturated, partially or completely halogenated hydrocarbon group, or
- an aryl group, which may be substituted with a 1-6 carbon atom straight or branched chain, saturated or partially or completely unsaturated hydrocarbon group, which, optionally, is partially or completely halogenated, or with a halogen atom, or
- a benzyl group that may contain substituents in the aromatic ring.

During the production method of compound **20** according to the invention compound **18** or its salt is reacted with compound **19** in the presence of a base, optionally in a further solvent, at a temperature between (- 20) - 50 °C, preferably between 0-25 °C, especially preferably between 0-5 °C, where in compound **19** R means
- a 1-6 carbon atom straight or branched chain saturated or partially or completely unsaturated hydrocarbon group, or
- a 1-6 carbon atom straight or branched chain saturated or partially or completely unsaturated, partially or completely halogenated hydrocarbon group, or
- an aryl group, which may be substituted with a 1-6 carbon atom straight or branched chain, saturated or partially or completely unsaturated hydrocarbon group, which, optionally, is partially or completely halogenated, or with a halogen atom
- a benzyl group that may contain substituents in the aromatic ring.

In formula **19** R means preferably a 1-6 carbon atom straight or branched chain saturated or partially or completely unsaturated hydrocarbon group, or benzyl group, more preferably a methyl, ethyl or benzyl group, especially preferably an ethyl group.

The base used is pyridine or a derivative of it, or an aliphatic amine, within this a straight or branched chain, cyclic tertiary or secondary amine, or salts of alkali metals formed with organic acids, preferably pyridine, diisopropylethylamine, triethylamine, morpholine, Na-acetate, diazabicyclooctane, especially preferably triethylamine or diisopropylethylamine

The solvent used is not especially limited, a suitable solvent is ethyl acetate, tetrahydrofuran, dichloromethane, acetone, acetonitrile, DMSO, DMF, preferably acetone or acetonitrile, especially preferably acetonitrile.

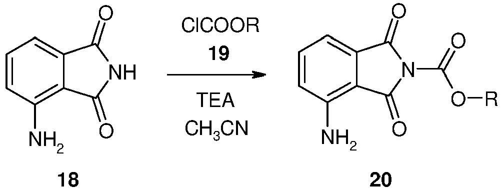

### Reaction scheme 5: the production of the compound of general formula 20

Compound **18** is a known, commercially available compound. Its structure contains an aniline-type NH₂ group (pKₐ= -25) displaying the character of a base and an NH group (pKₐ= 8-9) displaying the character of an acid amide.

It was surprising to experience that using the synthesis pathway developed by us (reaction scheme 5), under the reaction conditions used, compound **18** may be easily transformed into compound **20,** with good yield and high purity, while the basic, relatively more reactive NH₂ group remains intact in spite of the strong acylation agents (chloroformate esters) used and the proportion of the products **21** and **22** (reaction scheme 6) expected by a person skilled in the art does not reach 0.10% (HPLC) in product **20.**

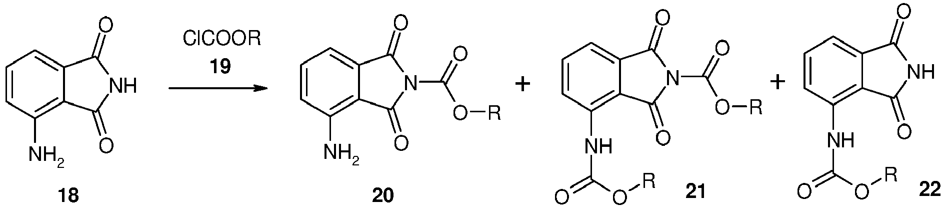

### Reaction scheme 6: the possible products of the reaction of compounds 18 and 19

The compound **20** according to the invention may be used as an intermediate for the production of pomalidomide **1** or its pharmaceutically acceptable salts, hydrates and cocrystals. Accordingly, the subject of the invention relates to a method for the production of formula **1** pomalidomide. During the method the compound of general formula **20** is reacted with glutamine, its open chain or cyclic derivative or the metal salt of any of these, preferably with compound **3** or its metal salt, optionally in the presence of an acid binder; or glutamin, with an acid addition salt of an open chain or cyclic derivative of it, preferably with the acid addition salt of compound **3,** especially preferably with its hydrochloride salt, in the presence of am acid binder, optionally in further solvent.

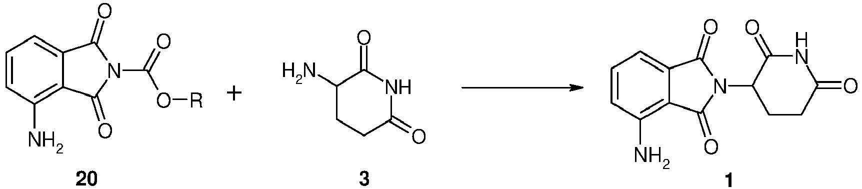

### Reaction scheme 7: the production of pomalidomide from compound 20

During the reaction the acid binder used is pyridine or its derivative, or an aliphatic amine, within this a straight or branched chain, cyclic tertiary or secondary amine, or a salt of alkali metals formed with organic acids, preferably pyridine, diisopropylethylamine, triethylamine, morpholine, Na-acetate, especially preferably triethylamine or Na-acetate.

The solvent used is not particularly limited, a suitable solvent may be a 1-4 carbon atom aliphatic alcohol, a 1-5 carbon atom straight chain or cyclic ether, a 1-5 carbon atom straight or brached chain ketone, a 1-6 carbon atom ester, acetonitrile, DMSO, DMF, water, or a mixture of these solvents, preferably methyl alcohol, ethyl alcohol, 2-propanol, THF, acetone, ethyl acetate, acetonitrile, DMSO, DMF, more preferably DMF or acetonitrile, especially preferably acetonitrile. The selection of the appropriate solvent belongs to the compulsory knowledge of a person skilled in the art.

The temperature used is between 0 °C and the reflux temperature, preferably between 50 °C and the reflux temperature.

In summary the method according to reaction scheme 8 presents the realisation of the objective of the invention:

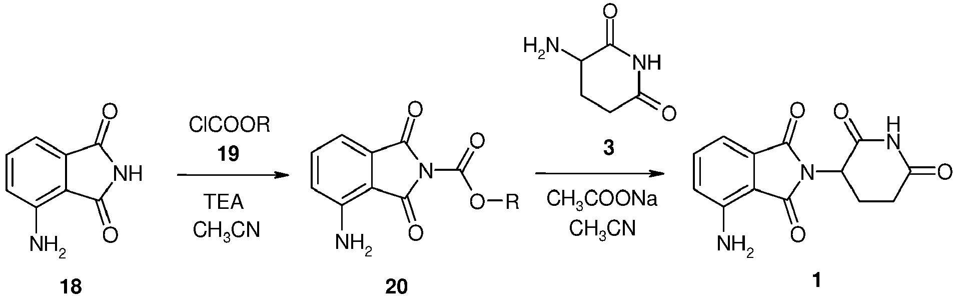

### Reaction scheme 8: The novel synthesis pathway of pomalidomide according to the present application

In summary it was surprising to experience that the novel synthesis pathway progressing through the novel intermediate according to the present application is preferable to (more effective and economic than) the reaction pathways known of in the literature. The yield relating to the production of compound **20** is 90.3%, which is coupled with an HPLC purity level of 99.70%. These same data for the raw, isolated pomalidomide of formula 1 are a yield of 96.6% and an HPLC purity level of 99.90%, which easily complies with pharmaceutical industry prescriptions. Accordingly the total yield from the two reaction steps is 87.2%, which result in well characterised pomalidomide Form A product with excellent purity values.

There is a frequent demand in pharmaceutical industry active substance production for the end product to have been subjected to filtering, for the purpose of removing any mechanical contaminants, and, via this, recrystallization. With the recrystallization described in the present application the purity of the end product pomalidomide may be increased further, which involves a slight drop in total yield (84.9%). From a crystal structure point of view the recrystallized end product is identical to the Form A described in international publication document number WO 2013126326.

The invention makes it possible to produce a pharmaceutical preparation containing a therapeutically effective amount of pomalidomide **(1)** produced with the method according to the invention, optionally with a pharmaceutically acceptable carrier. The invention also makes it possible to use the pomalidomide or its salts produced according to the invention as a medicine, or to use them for the production of a pharmaceutical preparation.

The pharmaceutical preparations containing the pomalidomide produced using the method according to the invention are preferably administered orally. Preparations that maybe administered orally include, for example, tablets, capsule, dragées, solutions, elixirs, suspensions or emulsions.

The pharmaceutical preparations containing the pomalidomide produced with the method according to the invention may contain the usual pharmaceutical carrier materials and/or excipients. Carriers include, for example, magnesium carbonate, magnesium stearate, talc, sugar, lactose, pectin, dextrin, starch, gelatine, tragacanth, methyl cellulose, sodium carboxymethyl cellulose, low melting point wax, PEG, cocoa butter, etc. In the case of capsules it is frequently the material of the capsule that serves as the carrier and so in such a case there is no need for a separate carrier. Orally administered preparations also include sachets and lozenges. Tablets, powders, capsules, pills, sachets and lozenges are especially suitable solid preparation forms.

The tablets may be produced by mixing the active substance with carrier materials with suitable characteristics in the appropriate ratio, and by pressing the desired shape and size tablets from the mixture.

Powders are produced by mixing the finely powdered active substance with finely powdered carrier material. Liquid preparations include solutions, suspensions and emulsions, from which, optionally, the active substance is released in a delayed way. Aqueous and aqueous propylene glycol solutions are preferable. Liquid preparations serving for parenteral administration may be preferably produced in the form of a propylene glycol solution.

The medical preparations containing the pomalidomide produced with the method according to the invention are preferably produced in the form of dose units. The dose units contain the desired amount of active substance. The dose units may be distributed in a packaged form, which contains separated amounts of the preparations (e.g. packaged tablets, capsules, powder in vials or ampules). The dose unit relates to the capsule, the tablet, sachet, lozenge as well as to the packaging containing a sufficient number of unit doses.

The invention also makes it possible to produce the above pharmaceutical preparations in such a way that the pomalidomide or one of its salts or a mixture of these produced according to the invention is mixed with pharmaceuically suitable solid or liquid diluents and/or excipients, and the mixture is placed into a galenic form.

The medical preparations mentioned above may be produced using the usual methods of pharmaceutical production. If necessary, these medical preparations may also contain further pharmaceutical active substances with the compounds or a mixture of compounds according to the invention.

The pomalidomide or any of its salts produced with the method according to the invention may be used for the production of a pharmaceutical preparation serving for the treatment of cancer diseases.

The pomalidomide or any of its salts produced according to the invention may therefore be used in the treatment of cancer diseases. The cancer disease may be, among others, multiple myeloma.

Further details of the solution according to the invention are presented in the following examples without restricting the scope of protection of the invention to these examples.

### FIGURES

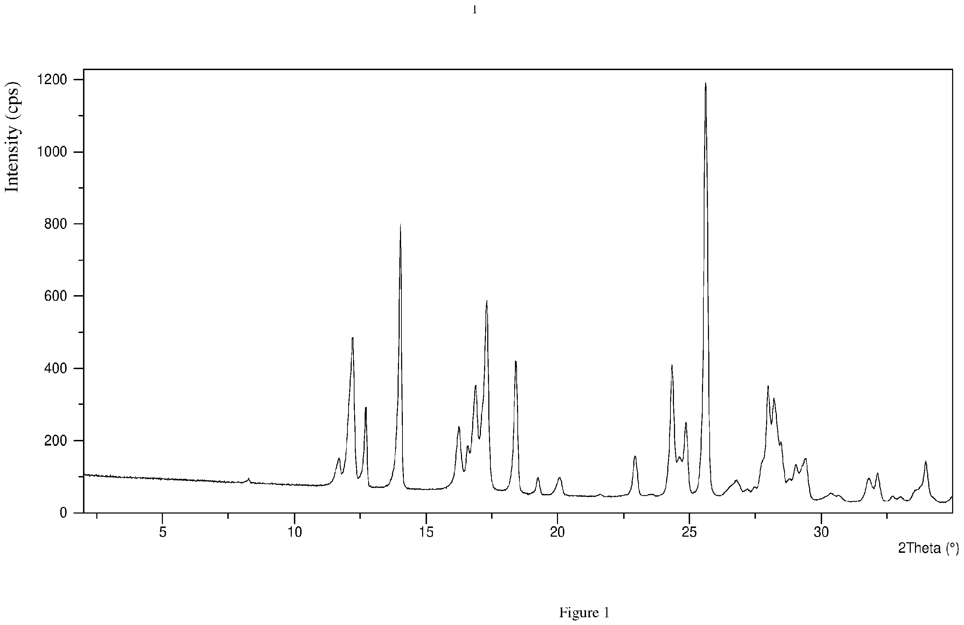

**Figure 1****:** The x-ray powder diffractogram of the formula 1 pomalidomide base Form A

### EXAMPLES

The following instruments, settings and methods were used in order to characterise the components obtained in the examples.

### X-ray powder diffractogram measurement conditions

| | |
|---|---|
| Device: | PANalytical Empyrean X-ray powder diffractometer |
| Measurement mode: | Transmission |

| X-rav tube | |
|---|---|
| Type: | Empyrean Long Fine Focus High Resolution tube |
| Anode: | Cu |
| Wavelength: | Kα (1.541874 Å) |
| Focussing: | line focus |

### Radiation side optics

| | |
|---|---|
| Divergence slit: | Fixed slit 1/2 ° |
| Mirror: | Elliptic focussing mirror |
| Soller slit: | 0.04 rad |
| Diffusion inhibitor slit: | Fixed slit 1/2 ° |

### Detector side optics

| | |
|---|---|
| Diffusion inhibitor slit: | Programmable slit, in fixed mode: 1/2 ° |
| Soller slit: | 0.04 rad |

### Sample table

| | |
|---|---|
| Type: | Reflection-transmission spinner stage |
| Sample rotation rate: | 1 rps |
| Beam knife: | Transmission |

### Detector

| | |
|---|---|
| Type: | PIXcel 3D 1×1 area detector |
| Detecting mode: | Scanning line detector (ID) operation mode |
| Active length: | 3.3473° |
| Sample preparation: | unpulverised samples placed between Mylar sheets |

### Measurement conditions:

| | |
|---|---|
| Temperature: | room temperature |
| Accelerating voltage: | 45 kV |
| Anode current: | 40 mA |
| Recording type: | continuous (θ/θ) scanning |
| Measurement range: | 2.0000 - 34.9964° 2θ |
| Step gap: | 0.0131° 2θ |
| Counts measurement time: | 109.650 s |
| Number of measurement cycles: | 1 |
| Measurement time: | -20 minutes |

### NMR recordings

The ¹H- and ¹³C-NMR spectra were recorded in a DMSO-*d₆* solvent, using a Bruker Avance III (400 MHz in the case of ¹H-NMR and approximately 100 MHz in the case of ¹³C-NMR) spectrometer in the presence of TMS, as internal standard.

### Example 1

### Ethyl 4-amino-1,3-dioxo-1,3-dihydro-2H-isoindole-2-carboxylate (20, R = Et)

60.0 g (370.0 mmol) of 3-aminophthalimide **(18),** 900 cm³ of acetonitrile, 93.6 g (925.0 mmol) triethylamine are measured into a 2000 cm³ round-bottomed flask, then the suspension obtained in this way is cooled to 0 °C. While stirring a solution made of 60.23 g (555.0 mmol) of ethyl chloroformate **(19,** R = Et) with 120 cm³ of acetonitrile is added over the course of 60 minutes, whilst maintaining the temperature between 0-5 °C the stirring is continued at this temperature for 1 to 2 hours. The reaction mixture is left to warm up to room temperature and the stirring is continued for 1 hour. Using a vacuum the reaction mixture is concentrated to 200 cm³, then a solution of 3.0 cm³ cc. hydrochloric acid made with 900 cm³ of distilled water is added to it. The suspension is again cooled to 0 to 5 °C and then stirred at this temperature for 30 to 60 minutes. The crystalline product is filtered, washed with 2×120 cm³ of solvent mixture (acetonitrile/water = 1/4), then dried in a vacuum at 50 °C until constant weight is achieved. In this way 78.28 g (90.3%) of the product according to the title is obtained, which is taken to the next reaction without purification.

Mp.: 158-159 °C

IR (KBr): 3457, 3355, 1783, 1758, 1640, 1169 cm⁻¹.

¹H NMR (DMSO-*d*₆, 400 MHz): δ = 7.52 (m, 1H), 7.06 (m, 1H), 7.03 (m, 1H), 6.72 (b, 2H), 4.33 (q, J=7.1 Hz, 2H), 1.32 (t, J=7.1 Hz, 3H) ppm.

HPLC: Waters Acquity UPLC BEH C18; 2.1×50 mm; 1.7 µm; 0.5 mL/min; 225 nm; 10/90 CH₃CN/0.1% HClO₄ (aq); 1.90 min (99.70%).

### Example 2

### The production of (R/S)-4-amino-2-(2,6-dioxopiperidin-3-yl)-1H-isoindole-1,3(2H)-dione (1)

40.0 g (170.8 mmol) of ethyl 4-amino-1,3-dioxo-1,3-dihydro-2*H*-isoindole-2-carboxylate, 800 cm³ of acetonitrile, 28.4 g (342.0 mmol) of anhydrous sodium acetate, 28.1 g (170.7 mmol) of 3-aminopiperidine-2,6-dione are measured into a 1000 cm³ round-bottomed flask, then the suspension obtained in this way is heated to reflux temperature. The stirring is continued at unchanged temperature for 4 hours. The reaction mixture is concentrated to about 40 cm³ at 40 °C with the help of a vacuum. 800 cm³ of distilled water is added to the concentrated residue, which is then stirred at room temperature for 30 minutes. (The water may also be added at an earlier stage.) Following this the crystalline product is filtered, washed with 2×400 cm³ of distilled water, then dried at 50 °C in a vacuum until constant weight is achieved. In this way 45.10 g (96.6%) of the product according to the title is obtained.

Mp.: 314-315 °C (decomposes)

IR (KBr): 3481, 3378, 3248, 1752, 1703, 1635, 1362, 1197 cm⁻¹.

¹H NMR (DMSO-*d*₆, 400 MHz): δ = 11.10 (b, 1H), 7.47 (m, 1H), 7.02 (m, 1H), 7.00 (m, 1H), 5.06 (m, 1H), 2.89 (m, 1H), 2.58 (m, 1H), 2.56 (m, 1H), 2.03 (m, 1H) ppm.

HPLC: Waters Acquity UPLC BEH C18; 2.1×50 mm; 1.7 µm; 0.5 mL/min; 225 nm; 10/90 CH₃CN/0.1% HClO₄ (aq); 1.38 min (99.90%).

### Example 3

### The recrystallization of (R/S)-4-amino-2-(2,6-dioxopiperidin-3-yl)-1H-isoindole-1,3(2H)-dione (1)

38.0 g (139.1 mmol) of the 4-amino-2-(2,6-dioxopiperidin-3-yl)-1*H*-isoindole-1,3(2*H*)-dione produced according to example 2 and 190 cm³ of DMSO is measured into a 2000 cm³-round-bottomed flask, then the suspension obtained in this way is heated to 50 °C and stirred at this temperature until completely dissolved. 190 cm³ of acetone is added to the clear solution, then the mixture is cooled to room temperature, filtered if necessary, and 760 cm³ of distilled water is added over the course of 30 minutes. The suspension created is cooled to 0 to 5 °C and then stirred for 30 to 60 minutes. The crystalline product is filtered cold, washed with 100 cm³ of acetone, then dried in a vacuum at 50 °C until constant weight is achieved. In this way 36.97 g (97.3%) of the Form A product according to the title is obtained.

Mp.: 316-318 °C (decomposes)

IR (KBr): 3481, 3378, 3248, 1752, 1703, 1635, 1362, 1197 cm⁻¹.

¹H NMR (DMSO-*d*₆, 400 MHz): δ = 11.10 (b, 1H), 7.47 (m, 1H), 7.02 (m, 1H), 7.00 (m, 1H), 5.06 (m, 1H), 2.89 (m, 1H), 2.58 (m, 1H), 2.56 (m, 1H), 2.03 (m, 1H) ppm.

HPLC: Waters Acquity UPLC BEH C18; 2.1×50 mm; 1.7 µm; 0.5 mL/min; 225 nm; 10/90 CH₃CN/0.1% HClO₄ (aq); 1.38 min (99.95 %).

XRPD: The x-ray powder diffractogram of the sample may be seen in annex 1, which correlates to the Form A described in international application number WO2013126326.

## Claims

1. Method for the production of the compound of general formula **20,** 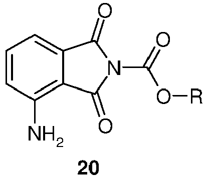 where in the formula R means
- a 1-6 carbon atom straight or branched chain, or 3-6 carbon atom cyclic, saturated or partially or completely unsaturated hydrocarbon group, or
- a 1-6 carbon atom straight or branched chain, or 3-6 carbon atom cyclic, saturated or partially or completely unsaturated, partially or completely halogenated hydrocarbon group, or
- an aryl group, which may be substituted with a 1-6 carbon atom straight or branched chain, saturated or partially or completely unsaturated hydrocarbon group, which, optionally, is partially or completely halogenated, or with a halogen atom, or
- a benzyl group that may contain substituents in the aromatic ring
and its salts, **characterised by** that compound **18** 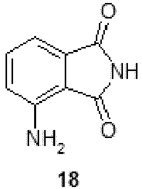 or its acid addition salt is reacted with compound **19**
ClCOOR **19**
in the presence of a base; or a metal salt of compound **18** is reacted with compound **19** optionally in the presence of a base, optionally in a further solvent at a temperature between (- 20) - 50 °C, preferably between 0-25 °C, especially preferably between 0-5 °C, where in compound **19** R means
- a 1-6 carbon atom straight or branched chain, saturated or partially or completely unsaturated hydrocarbon group,
- a 1-6 carbon atom straight or branched chain, saturated or partially or completely unsaturated, partially or completely halogenated hydrocarbon group, or
- an aryl group, which may be substituted with a 1-6 carbon atom, straight or branched chain, saturated or partially or completely unsaturated hydrocarbon group, which, optionally, is partially or completely halogenated, or with a halogen atom, or
- a benzyl group that may contain substituents in the aromatic ring.

2. Method according to claim 1, **characterised by** that the reaction is performed with compound **19**
ClCOOR 19
where in the formula R means a 1-6 carbon atom straight or branched chain, saturated, or partially or completely unsaturated hydrocarbon group, or benzyl group, preferably a methyl, ethyl or benzyl group, especially preferably an ethyl group.

3. The method according to claims 1 to 2, **characterised by** that the base used is pyridine or a derivative of it, or an aliphatic amine, within this a straight or branched chain, cyclic tertiary or secondary amine, or salts of alkali metals formed with organic acids, preferably pyridine, diisopropylethylamine, triethylamine, morpholine, Na-acetate, diazabicyclooctane, especially preferably triethylamine or diisopropylethylamine.

4. Method for the production of pomalidomide of formula **1** 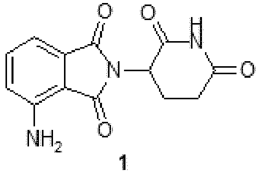 or its pharmaceutically acceptable salts, hydrates and cocrystals, **characterised by** that the compound of general formula **20** 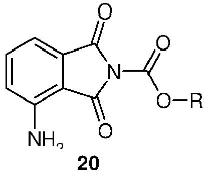 is reacted with glutamine, its open chain or cyclic derivative or the metal salt of any of these, preferably with compound **3** 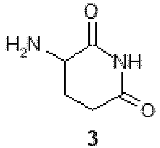 or its metal salt, optionally in the presence of an acid binder; or glutamine, with an acid addition salt of an open chain or cyclic derivative of it, preferably with the acid addition salt of compound **3,** in the presence of an acid binder, optionally in a further solvent and then recrystallized as necessary, whereas the substituent R is defined according to claim 1.

5. Method according to claim 4, **characterised by** that the compound of general formula **20** 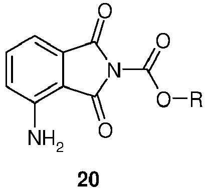 is reacted with the hydrochloride salt of compound **3**

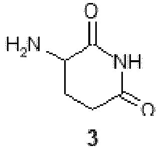

6. Method according to claims 4 to 5, **characterised by** that the acid binder used is pyridine or its derivative, or an aliphatic amine, within this a straight or branched chain, cyclic tertiary or secondary amine, or a salt of alkali metals formed with organic acids, preferably pyridine, diisopropylethylamine, triethylamine, morpholine, Na-acetate, especially preferably triethylamine or Na-acetate.

## Patentansprüche

1. Verfahren zur Herstellung der Verbindung der allgemeinen Formel 20, 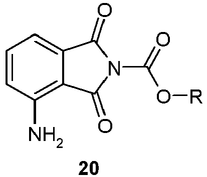 wobei R ausgewählt ist aus Bedeutungen
- eine geradkettige oder verzweigte Kohlenwasserstoffgruppe mit 1 bis 6 Kohlenstoffatomen oder eine cyclische, gesättigte oder teilweise oder vollständig ungesättigte Kohlenwasserstoffgruppe mit 3 bis 6 Kohlenstoffatomen, oder
- eine geradkettige oder verzweigte Kohlenwasserstoffgruppe mit 1 bis 6 Kohlenstoffatomen oder eine cyclische, gesättigte oder teilweise oder vollständig ungesättigte, teilweise oder vollständig halogenierte Kohlenwasserstoffgruppe mit 3 bis 6 Kohlenstoffatomen oder
- eine Arylgruppe, die mit einer geradkettigen oder verzweigten, gesättigten oder teilweise oder vollständig ungesättigten Kohlenwasserstoffgruppe mit 1-6 Kohlenstoffatomen, die gegebenenfalls teilweise oder vollständig halogeniert ist, oder mit einem Halogenatom substituiert sein kann, oder
- eine Benzylgruppe, die Substituenten im aromatischen Ring enthalten kann
und deren Salze, **dadurch gekennzeichnet, dass** die Verbindung 18 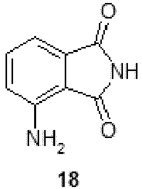 oder deren Säureadditionssalz mit Verbindung 19 umgesetzt wird
CICOOR **19**
- a 1-6 carbon atom straight or branched chain, saturated or partially or completely unsaturated hydrocarbon group,
in Gegenwart einer Base umgesetzt wird; oder ein Metallsalz der Verbindung 18 mit der Verbindung 19 gegebenenfalls in Gegenwart einer Base, gegebenenfalls in einem weiteren Lösungsmittel bei einer Temperatur zwischen (- 20) - 50 °C, vorzugsweise zwischen 0-25 °C, besonders bevorzugt zwischen 0-5 °C, umgesetzt wird, wobei in der Verbindung 19 R bedeutet
- eine geradkettige oder verzweigte, gesättigte oder teilweise oder vollständig ungesättigte Kohlenwasserstoffgruppe mit 1 bis 6 Kohlenstoffatomen,
- eine gerad- oder verzweigtkettige, gesättigte oder teilweise oder vollständig ungesättigte, teilweise oder vollständig halogenierte Kohlenwasserstoffgruppe mit 1-6 Kohlenstoffatomen oder
- eine Arylgruppe, die mit einer gerad- oder verzweigtkettigen, gesättigten oder teilweise oder vollständig ungesättigten Kohlenwasserstoffgruppe mit 1-6 Kohlenstoffatomen, die gegebenenfalls teilweise oder vollständig halogeniert ist, oder mit einem Halogenatom substituiert sein kann, oder
- eine Benzylgruppe, die Substituenten im aromatischen Ring enthalten kann.

2. Verfahren nach Anspruch 1, **dadurch gekennzeichnet, dass** die Reaktion mit Verbindung 19 durchgeführt wird.
ClCOOR **19**
wobei R ausgewählt ist aus Bedeutungen eine geradkettige oder verzweigte, gesättigte oder teilweise oder vollständig ungesättigte Kohlenwasserstoffgruppe mit 1-6 Kohlenstoffatomen oder eine Benzylgruppe, vorzugsweise eine Methyl-, Ethyl- oder Benzylgruppe, besonders bevorzugt eine Ethylgruppe.

3. Verfahren nach einem der Ansprüche 1 bis 2, **dadurch gekennzeichnet, dass** als Base Pyridin oder ein Derivat davon oder ein aliphatisches Amin, darin ein gerad- oder verzweigtkettiges, cyclisches tertiäres oder sekundäres Amin, oder mit organischen Säuren gebildete Salze von Alkalimetallen, vorzugsweise Pyridin, Diisopropylethylamin, Triethylamin, Morpholin, Na-Acetat, Diazabicyclooctan, besonders bevorzugt Triethylamin oder Diisopropylethylamin, verwendet wird.

4. Verfahren zur Herstellung der Formel 1 Pomalidomid 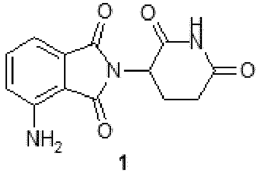 oder eines pharmazeutisch verträglichen Salzes, Hydrates oder Ko-kristalles, **dadurch gekennzeichnet, dass** die Verbindung der allgemeinen Formel 20 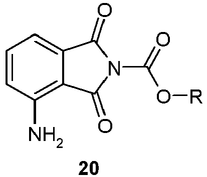 mit Glutamin, seinem offenkettigen oder cyclischen Derivat oder dem Metallsalz eines dieser Derivate, vorzugsweise mit Verbindung 3, umgesetzt wird 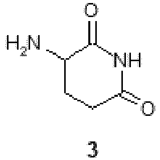 oder seines Metallsalzes, gegebenenfalls in Gegenwart eines sauren Bindemittels; oder Glutamin mit einem Säureadditionssalz eines offenkettigen oder cyclischen Derivats davon, vorzugsweise mit dem Säureadditionssalz der Verbindung 3, in Gegenwart eines sauren Bindemittels, gegebenenfalls in einem weiteren Lösungsmittel und dann erforderlichenfalls umkristallisiert, wobei der Substituent nach Anspruch 1 definiert ist.

5. Verfahren nach Anspruch 4, **dadurch gekennzeichnet, dass** die Verbindung der allgemeinen Formel 20 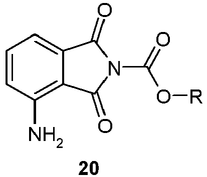 mit dem Hydrochloridsalz von Verbindung 3 umgesetzt wird

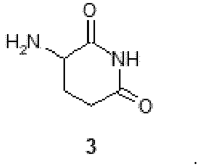

6. Verfahren gemäß einem der Ansprüche 4 oder 5, **dadurch gekennzeichnet, dass** als saures Bindemittel Pyridin oder dessen Derivat, oder ein aliphatisches Amin, darin ein gerad- oder verzweigtkettiges, cyclisches tertiäres oder sekundäres Amin, oder ein mit organischen Säuren gebildetes Alkalisalz, vorzugsweise Pyridin, Diisopropylethylamin, Triethylamin, Morpholin, Na-Acetat, besonders bevorzugt Triethylamin oder Na-Acetat, erhalten wird.

## Revendications

1. Méthode de production du composé de formule générale **20** 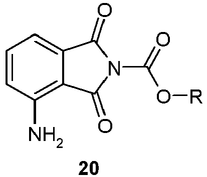 dans laquelle la formule R représentes
- une chaîne droite ou ramifiée de 1 à 6 atomes de carbon, ou un groupe hydrocarboné cyclique de 3 à 6 atomes de carbon saturé ou partiellement ou totalement insaturé, ou
- une chaîne droite ou ramifiée de 1 à 6 atomes de carbon, ou un groupe hydrocarboné cyclique de 3 à 6 atomes de carbon saturé ou partiellement ou totalement insaturé, partiellement ou totalement halogéné, ou
- un groupe aryle, qui peut être substitué par une chaîne droite ou ramifiée de 1 à 6 atomes de carbon, un groupe hydrocarboné saturé ou partiellement ou totalement insaturé, éventuellement partiellement ou totalement halogéné, ou par un atome d'halogène, ou
- un groupe benzyle, qui peut contenir des substituants dans le cycle aromatique
et les sels de celui-ci, **caractérisé en ce que** le composé **18** 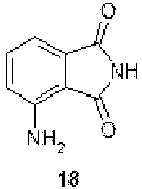 ou son sel d'addition d'acide est mis à réagir avec le composé **19**
ClCOOR **19**
en présence d'une base; ou un sel métallique du composé **18** est mis à réagir avec le composé **19** éventuellement en présence d'une base, éventuellement dans un autre solvent à une température entre (-20) - 50 °C, de préférence entre 0 - 25 °C, de manière particulièrement préférée entre 0 - 5 °C, dans laquelle le composé **19** représentes
- une chaîne droite ou ramifiée de 1 à 6 atomes de carbon, un groupe hydrocarbon saturé ou partiellement ou totalement insaturé, ou
- une chaîne droite ou ramifiée de 1 à 6 atomes de carbon, un groupe hydrocarbon saturé ou partiellement ou totalement insaturé, partiellement ou totalement halogéné, ou
- un groupe aryle, qui peut être substitué par une chaîne droite ou ramifiée de 1 à 6 atomes de carbon, un groupe hydrocarboné saturé ou partiellement ou totalement insaturé, éventuellement partiellement ou totalement halogéné, ou par un atome d'halogène, ou
- un groupe benzyle, qui peut contenir des substituants dans le cycle aromatique.

2. Méthode selon la revendication 1, **caractérisé en ce que** la réaction est effectuée avec le composé **19**
ClCOOR **19**
dans laquelle la formule R représentes une chaîne droite ou ramifiée de 1 à 6 atomes de carbon, un groupe hydrocarboné saturé ou partiellement ou totalement insaturé, ou un groupe benzyle, de préférence un groupe méthyle, éthyle ou benzyle, de manière particulièrement préférée un group éthyle.

3. Méthode selon les revendications 1 à 2, **caractérisé en ce que** la base utilisée est la pyridine ou un dérivé de celle-ci, ou une amine aliphatique dans ce une chaîne droite ou ramifiée, une amine cyclique tertiaire ou secondaire, ou des sels de métaux d'alcaline formés avec des acides organiques, de préférence la pyridine, la diisopropyléthylamine, la triéthylamine, la morpholine, Na-acétate, diazabicyclooctane, de manière particulièrement préférée la triéthylamine ou la diisopropylethylamine.

4. Méthode de production de pomalidomide de la formule **1** 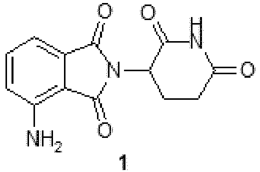 ou ses sels, hydrates et cocrystaux pharmaceutiquement acceptable, **caractérisé en ce que** le composé de formule générale **20** 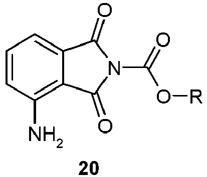 est mis à réagir avec la glutamine, sa chaîne ouverte ou son dérivé cyclique ou le sel métallique de l'un quelconque de ceux-ci, de préférence avec le composé **3** 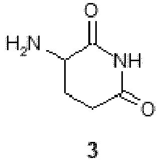 ou son sel métallique, éventuellement en présence d'un liant acide, ou la glutamine, avec un sel d'addition d'acide d'une chaîne ouverte ou un dérivé cyclique de celui-ci, de préférence avec le sel d'addition d'acide du composé **3,** en présence d'un liant acide, éventuellement dans un autre solvent, puis recristallisé si nécessaire, tandis que le substituant est défini selon la revendication 1.

5. Méthode selon la revendication 4, **caractérisé en ce que** le composé de formule Générale **20** 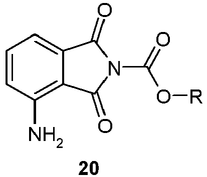 est mis à réagir avec le sel chlorhydrate du composé **3**

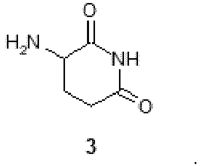

6. Méthode selon les revendications 4 à 5, **caractérisé en ce que** le liant acide utilisé est la pyridine ou son dérivé, ou une amine aliphatique dans ce une chaîne droite ou ramifiée, une amine cyclique tertiaire ou secondaire, ou un sel de métaux d'alcaline formés avec des acides organiques, de préférence la pyridine, la diisopropyléthylamine, la triéthylamine, la morpholine, Na-acétate, de manière particulièrement préférée la triéthylamine ou Na-acétate.
